# EUROPÄISCHE PATENTSCHRIFT

(11) **EP 0 604 748 B1**
(45) Veröffentlichungstag und Bekanntmachung des Hinweises auf die Patenterteilung: **14.02.2001**
(21) Anmeldenummer: 93118524.3
(22) Anmeldetag: 17.11.1993
(51) Int. Cl.: A61B 17/34

(54) **Trokarhülse**
Trocar sleeve
Manchon de trocart

(30) Priorität: 24.11.1992 DE 4239403
(43) Veröffentlichungstag der Anmeldung: 06.07.1994
(73) Patentinhaber: Karl Storz GmbH & Co. KG, 78532 Tuttlingen (DE)
(72) Erfinder: Storz, Karl, Dr. med. h.c., (DE)
(74) Vertreter: Weller, Wolfgang, Dr.rer.nat.

(56) Entgegenhaltungen:
- EP-A- 0 424 002
- EP-A- 0 589 412
- DE-A- 4 312 147

## Beschreibung

Die Erfindung betrifft eine Trokarhülse für endoskopische Operationen, mit einem Ventil und einer Dichtung am patientenfernen Ende der Trokarhülse, durch die Instrumente hindurchführbar sind, wobei die Trokarhülse, neben einer Dichtung mit großem Durchmesser, zumindest ein Trägerelement mit einer weiteren Dichtung mit kleinerem Durchmesser aufweist.

Eine derartige Trokarhülse ist aus dem Dokument EP-A-0 421 002 bekannt.

Je nach Art des Eingriffes kommen verschieden starke Instrumente zur Anwendung, die jeweils gasdicht gehandhabt werden müssen. Deshalb ist es erforderlich, eine meist aus Gummi bestehende Dichtung vorzusehen, meist in Form von Dichtungskappen, die verschiedene Bohrungen aufweisen, die dem Durchmesser bzw. der Stärke des jeweils einzuführenden Instrumentes entsprechen. Wird beispielsweise eine Dichtungskappe eingesetzt, die für ein Instrument mit einem relativ großen Außendurchmesser eingesetzt werden soll, weist die entsprechende Öffnung in der Dichtungskappe einen relativ großen Durchmesser auf. Würde nun durch eine solche Dichtung ein Instrument mit einem wesentlich geringeren Durchmesser eingeschoben werden, wäre ein dichtender Abschluß zwischen Dichtring und dem Instrument nicht mehr gewährleistet. Daher ist es erforderlich, verschiedene Dichtungen bzw. Gummikappen mit verschiedenen Bohrungen bereitzuhalten, um sie, falls erforderlich, auszuwechseln.

Dies ist zeitraubend und für den Operateur störend.

Bei der eingangs genannen, bekannten Trokarhülse ist das zumindest eine Trägerelement für die weitere Dichtung als separates Bauteil ausgebildet, das, im Bedarfsfalle, auf das patientenferne Ende der Trokarhülse aufgesetzt bzw. aufgedrückt werden kann. Dies bedarf vom Operateur einer gewissen Aufmerksamkeit, so daß er während diesem Zeitpunkt anderen Vorqängen an der Operationsstelle nicht seine Aufmerksamkeit schenken kann.

Aus dem Dokument EP-A-0 113 520 ist eine nephroskopische Kanüle bekannt, bei der am patientenfernen Ende eine Dichtung vorgesehen ist. Die Dichtung ist über einen flexiblen Verbindungssteg mit einem Ring verbunden, der in einer äußeren Ringnut der Kanüle sitzt. Somit ist die Dichtung über den flexiblen Verbindungssteg gelenkig mit der Kanüle verbunden, und kann ein- bzw. ausgeschwenkt werden.

Aus der US-A-4 112 932 ist eine Trokarhülse bekannt, an deren patientenfernem Ende eine Scheibe angeordnet ist, die mehrere Dichtungen mit unterschiedlichem Durchmesser trägt. Die Scheibe ist drehbar an der Trokarhülse angebracht, wobei sich die Drehachse parallel und im Abstand zur Längsachse der Trokarhülse erstreckt. Die Dichtungen auf der Scheibe sind nun so angeordnet, daß durch Drehen der Scheibe jeweils eine Dichtung in koaxialer Ausrichtung mit der Trokarhülse gebracht werden kann.

Aus dem Dokument EP-A-0 589 412, das Stand der Technik nach Artikel 54(3) EPÜ ist, kann eine Adapterdichtung an eine laparoskopische Kanüle über einen Befestigungsring angebracht werden und steht, über einen flexiblen Streifen, radial von der Kanüle ab. Soll die Adapterdichtung mit kleinerem Durchmesser eingesetzt werden, kann diese aufgrund der Flexibilität des Materials des Streifens um eine undefinierte Achse, die etwa quer zur Längsachse der Kanüle verläuft, verschwenkt und in eine entsprechende konische oder trichterförmige Ausnehmung am patientenfernen Ende der Kanüle eingeschoben und dabei zentriert werden. Da der biegsame Streifen keine definiert vorbestimmte Abknickstelle bzw. Schwenkachse aufweist, ist die Position der Adapterdichtung nach dem Verschwenken mehr oder weniger zentrisch, und es bedarf daher durch eine konische Ausgestaltung entsprechender Zentrierhilfen oder entsprechende Aufmerksamkeit durch die Bedienperson.

Aus der DE-A-43 12 147 ist eine ähnliche Konstruktion mit einem seitlich abstehenden biegsamen Streifen bekannt, dessen äußeres entferntes Ende die Dichtung mit geringerem Durchmesser trägt.

Es ist daher Aufgabe der vorliegenden Erfindung, eine Trokarhülse der eingangs genannten Art so zu verbessern, daß diese Nachteile beseitigt werden, und eine rasch und einfach durchzuführende Auswechslung der Dichtung möglich ist.

Die Aufgabe wird durch die Merkmale des kennzeichnenden Teils des Anspruchs 1 gelöst.

Der Umsetz- oder Auswechselvorgang ist in Sekundenschnelle durchzuführen und bedarf keiner hohen Aufmerksamkeit, d.h., die Aufmerksamkeit kann beispielsweise nach wie vor dem endoskopischen Vorgang zugewendet werden. Dadurch, daß das Trägerelement über ein Achsengelenk, nachfolgend vereinfachend Gelenk genannt, mit der Trokarhülse verbunden ist, kann der Auswechselvorgang durch einen einfachen Verschwenkvorgang durchgeführt werden, was sehr einfach, rasch und sicher durchführbar ist.

Erfindungsgemäß ist das Gelenk an einem starren Halter angeordnet, der besonders vorteilhaft radial zur Trokarhülse angeordnet ist.

Diese Maßnahmen haben den Vorteil, daß das Trägerlement, falls die darauf sitzende Dichtung nicht benötigt wird, radial abstehend ist, und somit die in axialer Richtung durchgeführten Manipulationen in der Trokarhülse nicht behindert. So können, falls dies erforderlich ist, in raumsparender Weise auch mehrere radial abstehende Trägerelemente eingesetzt werden, die dann entsprechend umfänglich beabstandet abstehen. Werden beispielsweise neben einem Instrument mit einem relativ großen Durchmesser noch zwei weitere Instrumente kleineren, jedoch untereinander unterschiedlichen Durchmessers eingesetzt, so können auch zwei, beispielsweise umfänglich um 90° bis 120° winkelversetzt abstehende Trägerelemente vorgesehen seien, die dann jeweils eingeschwenkt und an Ort und Stelle gebracht werden können.

In einer weiteren Ausgestaltung der Erfindung weist das Trägerelement einen Stutzen auf, über den es in die Durchtrittsöffnung in der Dichtung mit dem großen Durchmesser einsetzbar ist.

Diese Maßnahme hat nun den erheblichen Vorteil, daß das Trägerelement über den Stutzen einfach und im Paßsitz in die große Öffnung der Dichtung eingesetzt werden kann.

In einer weiteren Ausgestaltung der Erfindung ist das Ventil über einen Bajonettverschluß verschlossen, wobei das Ventil eine Bajonettführung aufweist, in die ein Deckel eindrehbar ist. Dabei ist besonders bevorzugt, daß der Deckel das zumindet eine Trägerelement trägt.

Diese Maßnahme hat den Vorteil, daß zum einen das Ventil zum Reinigen sehr einfach geöffnet werden kann, und daß zum anderen durch eine einfache Bajonettverschlußbewegung gegebenenfalls auch ein Deckel mit einem anderen Trägerelement eingesetzt werden kann. Es können dadurch Trokarhülsen mit an sich gleichartiger Baugröße mit unterschiedlichen Deckeln, die dann entsprechend unterschiedliche Trägerelemente aufweisen, bereitgestellt werden.

In einer weiteren Ausgestaltung der Erfindung ist das Gelenk schwergängig ausgebildet, so daß ein seitlich verschwenktes Trägerelement in der jeweiligen Stellung verbleibt, und bei schrägen und abgekippten Stellungen der Trokarhülse nicht durch das Eigengewicht selbständig verschwenkt wird.

Die Erfindung wird nachfolgend anhand einiger ausgewählter Ausführungsbeispiele in Zusammenhang mit den Zeichnungen näher beschrieben und erläutert. Es zeigen:
- Fig. 1: eine Seitenansicht eines ersten Ausführungsbeispieles einer erfindungsgemäßen Trokarhülse, ohne ein Instrument,
- Fig. 2: eine der Fig. 1 entsprechende Ansicht, jedoch nach Einführung eines durchmessergroßen Operationsinstrumentes,
- Fig. 3: eine der Darstellung von Fig. 2 entsprechende Darstellung, jedoch nach Einführung eines Operationsinstrumentes mit einem kleineren Durchmesser,
- Fig. 4: eine der Darstellung von Fig. 1 entsprechende Seitenansicht eines weiteren Ausführungsbeispieles mit einem Bajonettverschluß,
- Fig. 5: eine der Fig. 4 entsprechende Darstellung, bei der ein Deckelteil des Bajonettverschlusses abgenommen ist,
- Fig. 6: das in Fig. 5 ersichtliche abgenommene Deckelteil in einer Betriebsstellung mit geöffneter Ventilklappe, und
- Fig. 7: eine der Betriebsstellung von Fig. 3 vergleichbare Darstellung des Deckelteils der Trokarhülse allein.

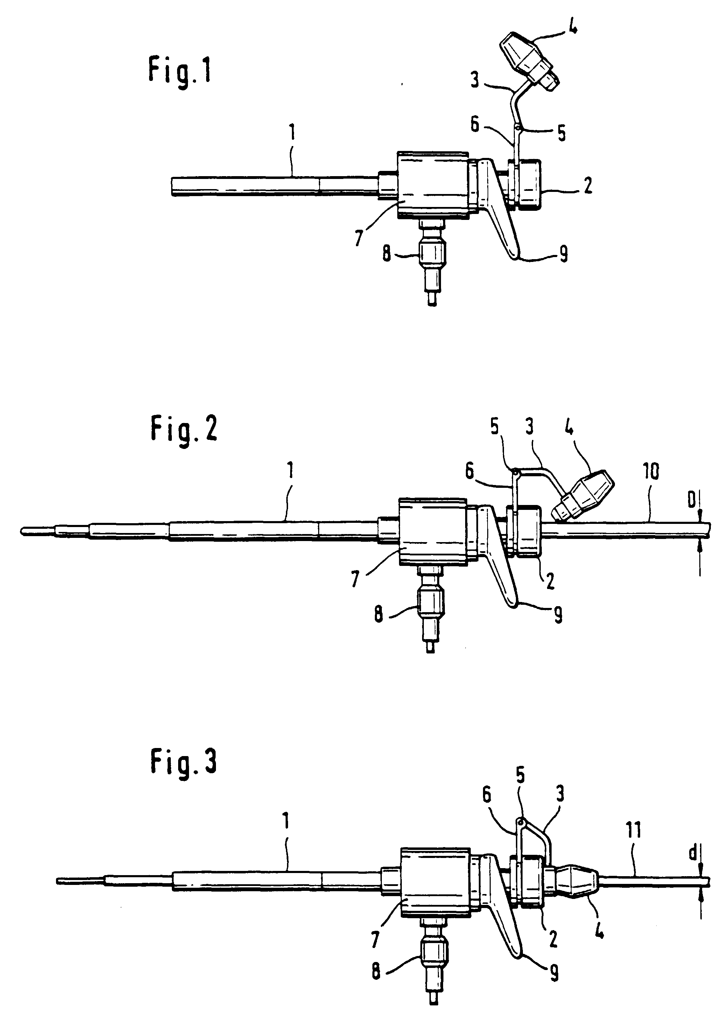

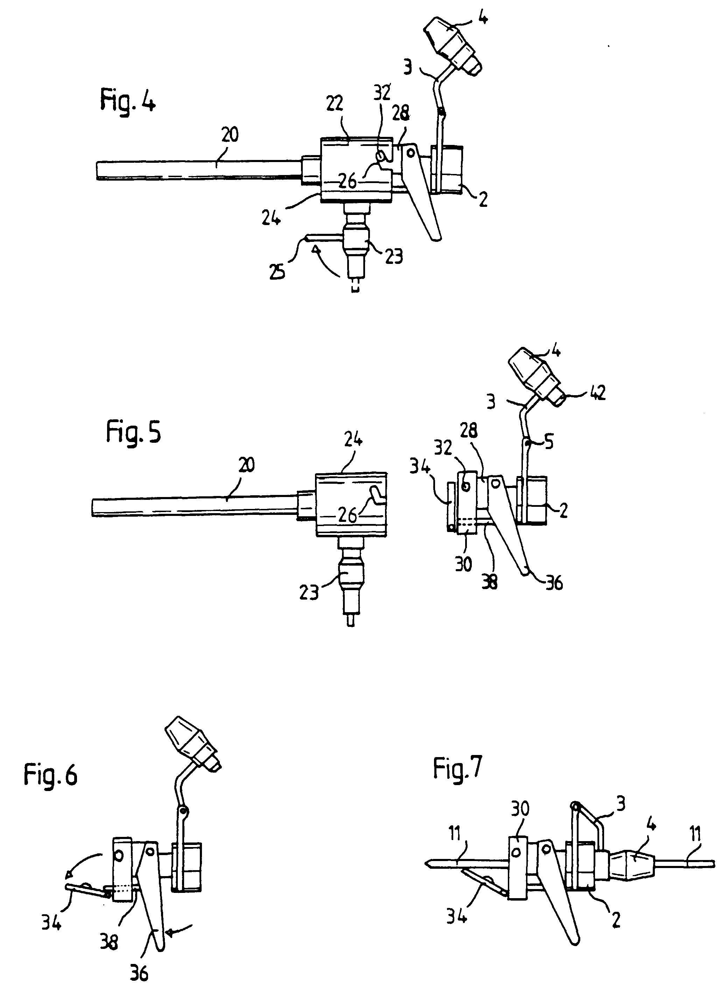

In Fig. 1 ist eine Trokarhülse 1 ersichtlich, an deren einem Ende ein Ventil 7 angeordnet ist. Bei dem Ventil 7 handelt es sich um ein Klappventil zum Durchlaß von Instrumenten mit unterschiedlicher Stärke. Die nähere Beschreibung eines solchen, an sich bekannten Ventils wird im Zusammenhang mit der Ausführungsform von Fig. 4 bis 7 durchgeführt.

Vom Ventil 7 steht radial ein Anschluß 8 für eine Spülflüssigkeit oder für den Luftdurchgang vor. Der Anschluß 8 kann über einen hier nicht näher bezeichneten Hahn geöffnet oder geschlossen werden. Derartige Anschlüsse mit verschiedenen Funktionen sind ebenfalls auf diesem Gebiet an sich bekannt und müssen daher nicht näher im einzelnen dargestellt werden.

Am patientenfernen Ende des Ventils 7 ist ein Hebel 9 zur Umstellung des Ventils 7 angeordnet.

Die nähere Funktionsweise und Ausgestaltung wird ebenfalls im Zusammenhang mit der Ausführungsform entsprechend den Fig. 4 bis 7 beschrieben.

Am patientenfernen Ende der Trokarhülse 1 ist eine kappenförmige Gummidichtung 2 vorgesehen, die zum Durchlaß für ein Instrument 10 mit einem großen Durchmesser D, wie das aus Fig. 2 zu ersehen ist, bestimmt ist.

Im Bereich der Gummidichtung 2 ist ein Halter 6 vorgesehen, der radial, d.h. in einem Winkel zu der Trokarhülse 1 stehend, angeordnet ist. Am äußeren Ende des Halters 6 ist ein Gelenk 5 vorgesehen, an dem ein Trägerelement 3 angeordnet ist, das mit einer Dichtungskappe 4 zum Durchlaß eines Operationsinstrumentes 11 mit kleinem Durchmesser d angeordnet ist, wie dies in Fig. 3 dargestellt ist.

In Fig. 1 ist eine Betriebsstellung dargestellt, in der das Trägerelement 3 mit der Dichtungskappe 4 um das Gelenk 5 radial nach außen gerichtet verschwenkt ist.

Aus Fig. 2 ist ersichtlich, daß ein Operationsinstrument 10 mit relativ großem Durchmesser D in die Trokarhülse eingeführt ist. Der Durchmesser D beträgt im dargestellten Ausführungsbeispiel etwa 5 mm. Das Operationsinstrument 10 reicht dabei in dichtendem Abschluß durch die Gummidichtung 2 bzw. durch die darin vorhandene, hier nicht näher bezeichnete Öffnung durch. Die Dichtungskappe 4 ist in der Darstellung von Fig. 2 mit dem Trägerelement 3 um das Gelenk 5 verschwenkt, die Handhabung des Operationsinstrumentes 10, das in axialer Richtung hin- und hergeschoben wird, ist dadurch nicht beeinträchtigt.

In Fig. 3 ist eine Situation dargestellt, bei der das Trägerelement 3 derart verschwenkt ist, daß einer in diesen Figuren nicht näher bezeichneter vorstehender Stutzen in die Öffnung der Gummidichtung 2 eingedrungen ist, so daß die Dichtungskappe 4 bzw. deren durchmessergeringe, hier nicht näher bezeichnete Durchtrittsöffnung koaxial auf der Trokarhülse sitzt. In Fig. 3 ist nun dargestellt, wie ein Operationsinstrument 11 mit einem kleineren Durchmesser d, hier beispielsweise 3 mm, in die Trokarhülse eingeschoben ist.

Es ist nun nicht notwendig, daß das Trägerelement 3 über den stutzenartigen Vorsprung in die Gummidichtung 2 eindringt, es ist auch ausreichend, wenn dieses lediglich an der Gummidichtung 2 anliegt.

Es ist einleuchtend, daß der Verschwenkvorgang aus der in Fig. 1 dargestellten Position über die in Fig. 2 dargestellte zu der in Fig. 3 dargestellten Verschwenkposition des Trägerelements 3 sehr einfach und in Sekundenschnelle durchzuführen ist.

Gleichermaßen rasch ist auch der umgekehrte Vorgang durchzuführen, d.h. nachdem aus der in Fig. 3 dargestellten Position das Operationsinstrument 11 abgezogen wurde, kann das Trägerelement 3 durch eine einfache Verschwenkbewegung wieder in die in Fig. 1 dargestellte Stellung verschwenkt werden, und es kann dann wieder durch die Dichtungskappe 2 ein Operationsinstrument 10 mit größerem Durchmesser eingeführt werden.

In den Fig. 1 bis 3 ist lediglich ein einziges Trägerelement 3 dargestellt, es ist aber auch möglich, beispielsweise zwei oder gar mehrere umfänglich verteilte Trägerelemente vorzusehen. Ferner kann vorgesehen sein, das Gelenk 5 so auszubilden, daß es relativ schwergängig ist, so daß ein seitlich verschwenktes Trägerelement jeweils in der Stellung verbleibt, und bei schrägen oder abgekippten Stellungen der Trokarhülse nicht durch das Eigengewicht selbständig verschwenkt wird. Andererseits ist die Schwergängigkeit nur derart, daß das Trägerelement 3 dennoch von Hand einfach bewegt werden kann.

Es ist auch die Möglichkeit vorgesehen, die Gummidichtung 2 zunächst abzunehmen, wobei dies durch ein Abziehen möglich ist, und erst dann das Trägerelement entsprechend zu verschwenken. Dabei wird dann dafür Sorge getragen, daß das Trägerelement dichtend an dem Ventil 7 sitzt.

In den Fig. 4 bis 7 ist eine weitere Ausführungsform dargestellt, die in ihren wesentlichen Bauelementen, insbesondere was das erfindungsgemäße Trägerelement 3 angeht, gleich wie die zuvor beschriebene Ausführung ausgebildet ist.

Die in den Fig. 4 und 5 dargestellte Trokarhülse 20 ist ebenfalls mit einem Ventil 22 versehen, von dem radial ein Anschluß 23 vorsteht. Ein Schwenkhebel 25 dient zum Öffnen und Schließen des Anschluß 23.

Das Ventil 22 weist ein Gehäuse 24 auf, an dessen patientenfernen Ende eine Bajonettführung 26 ausgespart ist.

Am patientenfernen Ende ist das Gehäuse 24 über einen Deckel 28 verschlossen, der an dem dem Gehäuse 24 zugewandten Ende mit einer Scheibe 30 versehen ist, deren Außendurchmesser in etwa dem Innendurchmesser des hohlzylindrischen Gehäuses 24 entspricht.

Von der Scheibe 30 steht radial ein Zapfen 32 vor, der zum Eingriff mit der Bajonettführung 26 vorgesehen ist.

An dem dem Gehäuse 24 zugewandten Ende ist die Scheibe 23 mit einer Ventilklappe 34 versehen, die auf verschiedene Art und Weisen geöffnet werden kann.

Aus Fig. 6 ist zu entnehmen, daß die Ventilklappe 34 dadurch geöffnet werden kann, daß ein Schwenkhebel 36 um seine hier nicht näher bezeichnete Schwenkachse verschwenkt wird, wie dies in Fig. 6 durch einen Pfeil dargestellt ist, wobei ein mit dem Schwenkhebel 36 in Verbindung stehender, axial ausgerichteter Stößel 38 die Ventilklappe 34 öffnet.

In Fig. 7 ist ersichtlich, daß ein Öffnen der Ventilklappe 34 auch durch ein durchgeschobenes Operationsinstrument 11 erfolgt (dies ist gleichermaßen auch bei einem durchmessergrößeren Instrument 10 der Fall).

Die in Fig. 7 dargestellte Verschwenkposition des Trägerelements 3 entspricht der in Fig. 3 dargestellten Position, d.h. ein vorstehender Stutzen 42 ist in die Dichtungskappe 4 eingerückt, so daß dann die Dichtungskappe 4 bzw. deren Durchtrittsöffnung koaxial mit der Durchtrittsöffnung der Gummidichtung 2 ausgerichtet ist.

## Patentansprüche

1. Trokarhülse für endoskopische Operationen, mit einem Ventil (7, 22) und einer Dichtung (2, 4) am patientenfernen Ende der Trokarhülse (1, 20), durch die Instrumente (10, 11) hindurchführbar sind, wobei die Trokarhülse (1, 20), neben einer Dichtung mit einem großen Durchmesser (2), zumindest ein Trägerelement (3) mit einer weiteren Dichtung (4) mit kleinerem Durchmesser aufweist, dadurch gekennzeichnet, daß das zumindest eine Trägerelement (3) starr ausgebildet ist und über ein Achsengelenk (5), das an einem starren Halter (6) angeordnet ist, mit der Trokarhülse (1) verbunden ist, wobei die Schwenkachse des Achsengelenkes (5) quer zur Längsachse der Trokarhülse (1, 20) verläuft, so daß die weitere Dichtung (4) eingeschwenkt werden kann.

2. Trokarhülse nach Anspruch 1, dadurch gekennzeichnet, daß der Halter (6) radial zur Trokarhülse (1) abstehend angeordnet ist.

3. Trokarhülse nach einem der Ansprüche 1 oder 2, dadurch gekennzeichnet, daß das Trägerelement (3) einen Stutzen (42) aufweist, über den es in die Durchtrittsöffnung in der Dichtung (2) mit großem Durchmesser einsetzbar ist.

4. Trokarhülse nach einem der Ansprüche 1 bis 3, dadurch gekennzeichnet, daß das Ventil (22) über einen Bajonettverschluß (26, 32) verschlossen ist.

5. Trokarhülse nach Anspruch 4, dadurch gekennzeichnet, daß das Ventil (22) ein eine Bajonettführung (26) aufweisendes Gehäuse (24) und einen darin einführbaren Deckel (28) aufweist.

6. Trokarhülse nach Anspruch 5, dadurch gekennzeichnet, daß der Deckel (28) das zumindest eine Trägerelement (3) trägt.

7. Trokarhülse nach einem der Ansprüche 1 bis 6, dadurch gekennzeichnet, daß das Gelenk (5) schwergängig ausgebildet ist, so daß ein seitlich verschwenktes Trägerelement (3) in der jeweiligen Stellung verbleibt.

## Claims

1. Trocar sleeve for endoscopic operations, comprising a valve (7, 22) and a seal (2, 4) at an end of the trocar sleeve (1, 20) remote from a patient, through which trovar sleeve instruments (10, 11) can be guided, the trocar sleeve (1, 20) has, besides a seal with a great diameter (2), at least one support element with a further seal (4) having a smaller diameter, characterized in that the at least one support element (33) is rigid and is connected to said trocar sleeve (1) via an axle articulation (5) provided at a rigid holder (6), the axis of articulation of said axle articulation (5) extends transverse to the longitudinal axis of the trocar sleeve (1, 20), so that the further seal (4) can be swivelled in.

2. Trocar sleeve according to claim 1, characterized in that the holder (6) is arranged radially extending from the trocar sleeve (1).

3. Trocar sleeve according to claims 1 or 2, characterized in that the support element (3) has a connecting piece (42) by means of which it can be inserted into a pass-through opening in the seal (2) with the large diameter.

4. Trocar sleeve according to anyone of claims 1 through 3, characterized in that the valve (22) is closed by a bayonet closure (26, 32).

5. Trocar sleeve according to claim 4, characterized in that that valve (22) comprises a housing (24) having a bayonet guide (26) and a cover (28) introducable therein.

6. Trocar sleeve according to claim 5, characterized in that the cover (28) supports that at least one support element (3).

7. Trocar sleeve according to anyone of claims 1 through 6, characterized in that the articulation (5) is designed tightly, so that a support element (3) swivelled laterally remains in its respective position.

## Revendications

1. Canule de trocart pour opérations endoscopiques, comportant une soupape (7, 22) et une garniture d'étanchéité (2, 4) à l'extrémité de la canule (1, 20) éloignée du patient, à travers laquelle des instruments (10, 11) peuvent être passés, la canule de trocart (1, 20) présentant, outre une garniture d'étanchéité de grand diamètre (2), au moins un élément porteur (3) avec une autre garniture d'étanchéité (4) de plus petit diamètre, caractérisée en ce que le ou les élément(s) porteur(s) (3) est (sont) réalisé(s) rigide(s) et est (sont) relié(s) à la canule de trocart (1), par une articulation à axe (5), qui est disposée sur un support (6) rigide, l'axe de pivotement de l'articulation à axe (5) s'étendant perpendiculairement à l'axe longitudinal de la canule de trocart (1, 20), de sorte que l'autre garniture d'étanchéité (4) peut pivoter vers l'intérieur.

2. Canule de trocart selon la revendication 1, caractérisée en ce que le support (6) est disposé de manière à dépasser radialement vers la canule de trocart (1).

3. Canule de trocart selon l'une des revendications 1 ou 2. caractérisée en ce que l'élément porteur (3) comporte une tubulure (42) par laquelle il peut être inséré dans l'orifice de passage pratiqué dans la garniture d'étanchéité (2) de grand diamètre.

4. Canule de trocart selon l'une des revendications 1 à 3, caractérisée en ce que la soupape (22) est fermée par une fermeture à baïonnette (26, 32).

5. Canule de trocart selon la revendication 4, caractérisée en ce que la soupape (22) présente un boîtier (24) qui comporte un guidage à baïonnette (26), ainsi qu'un couvercle (28) pouvant être introduit à l'intérieur de ce boîtier.

6. Canule de trocart selon la revendication 5. caractérisée en ce que le couvercle (28) supporte le ou les élément(s) porteur(s) (3).

7. Canule de trocart selon l'une des revendications 1 à 6. caractérisée en ce que l'articulation (5) est réalisée de manière à fonctionner difficilement, ce qui fait qu'un élément porteur (3) qui est pivoté sur le côté reste dans sa position.
